Europäisches Patentamt

European Patent Office

Office européen des brevets

(19)

(11) Numéro de publication: **0 334 815**
**A1**

(12) **DEMANDE DE BREVET EUROPEEN**

(21) Numéro de dépôt: **89810223.1**

(22) Date de dépôt: **21.03.89**

(51) Int. Cl.⁴: **G 21 K 3/00**
**H 05 K 9/00, A 61 N 1/16,**
**H 01 Q 15/00**

(30) Priorité: **21.03.88 CH 1057/88**

(43) Date de publication de la demande:
**27.09.89 Bulletin 89/39**

(84) Etats contractants désignés:
**AT BE DE ES FR GB GR IT LU NL SE**

(71) Demandeur: **PROGAM S.A.**
**La Lechere, c/o Denis Guignard**
**CH-1141 Yens (CH)**

(72) Inventeur: **Curchod, Jacques**
**Beaulieu 27**
**CH-1004 Lausanne (CH)**

(74) Mandataire: **Charbonnier, Georges R.**
**8, Avenue Peschier**
**CH-1206 Genève (CH)**

(54) **Filtre contre les radiations.**

(57) Le filtre contre les radiations est constitué par un treilli (10), en fil d'or, d'argent ou de cuivre de 0,1 mm, s'étendant dans un espace à trois dimensions.

Ce fil forme des figures géométriques à deux dimensions, en l'occurence des rectangles (11).

L'ensemble de ces figures constitue un réseau conducteur d'électricité que l'on peut charger électriquement ou mettre à terre par l'intermédiaire de deux bornes non représentées.

Fig.2

## Description

### FILTRE CONTRE LES RADIATIONS

Les écrans de télévision et d'ordinateurs , et la plupart des appareils électroniques sont des sources de rayonnement ( radio-activité, ondes de forme négatives, etc ) qui constituent un réel danger pour la santé des personnes de notre génération.

L'objet de la présente invention est un filtre destiné à absorber ces rayonnements en vue, sinon d'éliminer, tout au moins de réduire leurs effets nocifs.

Ce filtre, qui peut trouver des applications en biologie, en médecine, en agronomie notamment, est caractérisé par le fait qu'il comprend au moins un réseau de fils, conducteurs d'électricité ou non, reliés entre eux de manière à former des séries de figures géométriques élémentaires à deux ou trois dimensions et à trois ou quatre côtés.

Le dessin ci-annexé représente, schématiquement et à titre d'exemple, trois formes d'exécution de l'objet de l'invention ( représentation fragmentaire ).

La figure 1 est une vue en plan d'une première forme d'exécution comprenant un réseau de fils à deux dimensions.

Les figures 2 et 3 sont des vues en perspective de deux formes d'exécution comprenant chacun un réseau de fils à trois dimensions.

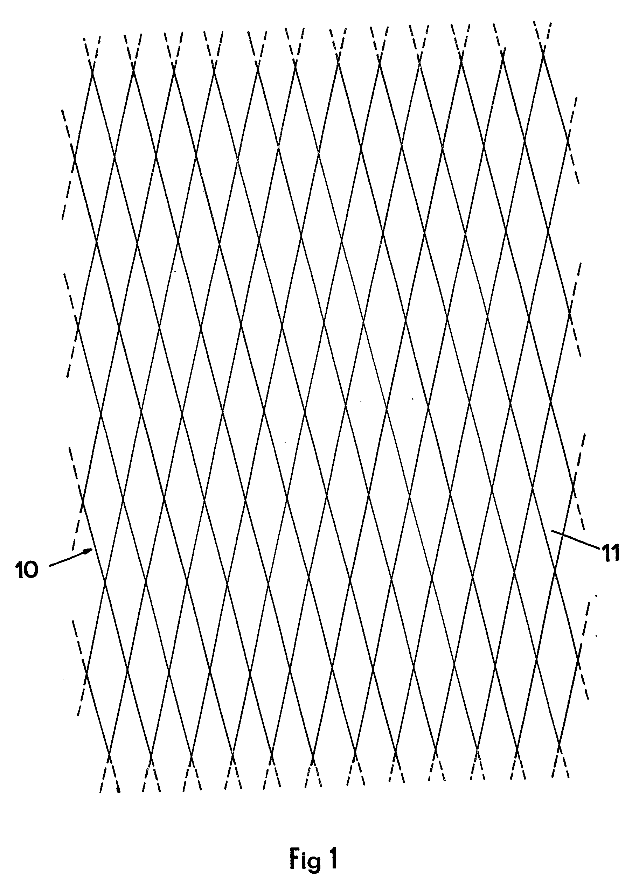

Le filtre représenté à la figure 1 est constitué par une grille 10, en fil d'or de 0,1 mm, dont les mailles forment des losanges 11. Deux bornes non représentées permettent soit de mettre le réseau à la terre pour le décharger, soit le relier à une source électrique pour le porter à un certain potentiel fixe ou variable.

Les dimensions des losanges sont très importantes car elles déterminent le spectre de fréquences des radiations absorbées.

On obtiendra un effet maximum en utilisant des mailles dont les diagonales sont comprises entre 4 et 7 mm, et 4 et 21 mm.

En superposant plusieurs filtres du type de celui représenté à la figure 1 dans lesquels les mailles sont constituées par des losanges 11 dont les petites diagonales ont des longueurs qui s'échelonnent graduellement de 4 à 7 mm, il est possible d'absorber pratiquement toutes les radiations comprises entre les rayons gamma et les rayons correspondant à la couleur noire ( spectre géobiologique ).

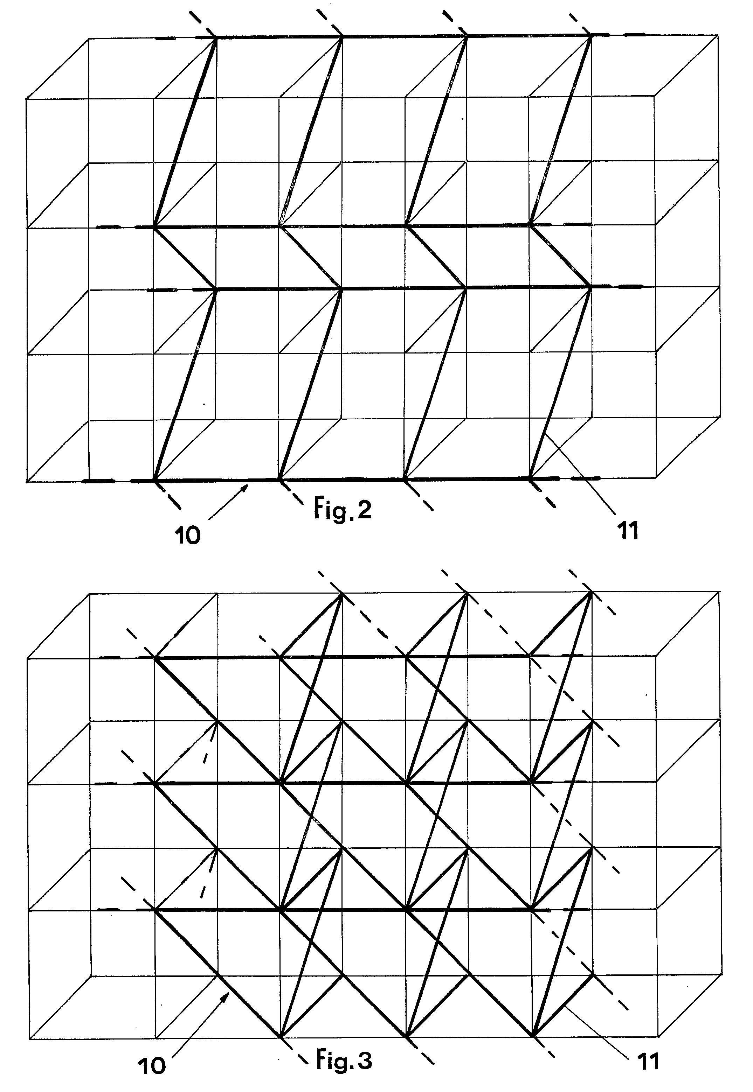

Les deux filtres représentés aux figures 2 et 3 sont constitués chacun par un treilli 10 , en fil d'or de 0,1 mm , s'étendant dans un espace à trois dimensions et formant des figures géométriques identiques 11 inscrites dans des cubes juxtaposés et superposés (cubes représentés en traits fins pour la compréhension du dessin).

Ces figures sont des rectangles dans le filtre de la figure 2, et des figures de forme relativement complexe dans le filtre de la figure 3.

Dans les deux filtres en question les fils du réseau constitue un circuit conducteur d'électricité qui peut être relié, comme le filtre de la figure 1, par des bornes non représentées à une prise de terre ou à une source électrique.

Les filtres à trois dimensions pourront se présenter sous forme de corps relativement plats de manière à pouvoir, comme les filtres à deux dimensions, être pliés, galbés ou cintrés pour s'adapter aux formes des sources, Ces filtres pourront éventuellement être montés sur des supports ad hoc, voir incorporés directement aux écrans de TV et d'ordinateurs.

Leur fabrication industrielle ne pose aucun problème et peut être réalisée en mettant en oeuvre soit des procédés classiques , soit des technologies modernes, par exemple la technique de fabrication des circuits imprimés.

L'invention n'est évidemment pas limitée aux formes d'exécution décrites à titre d'exemple.

En particulier les fils pourront être en argent, en cuivre, en laiton, constitués par des fibres optiques, etc. etc.

On précisera par ailleurs que les dimensions des figures composant les réseaux ne devront , en principe, pas dépasser 33 mm , le degré d'efficacité diminuant considérablement à partir de cette valeur.

## Revendications

1. Filtre contre les radiations, caractérisé par le fait qu'il comprend au moins un réseau (10) formé de fils, conducteurs d'électricité ou non, reliés entre eux de manière à former des séries de figures géométriques (11), à deux ou trois dimensions, et à trois ou quatre côtés.

2. Filtre selon la revendication 1, caractérisé par le fait que ledit réseau (10) est constitue par une grille s'étendant dans un espace à deux dimensions.

3. Filtre selon la revendication 2, caractérisé par le fait que lesdites figures (11) sont des losanges.

4. Filtre selon la revendication 3, caractérisé par le fait que les longueurs des diagonales des losanges sont comprises respectivement entre 4 et 21 mm et entre 4 et 7 mm.

5. Filtre selon la revendication 3, caractérisé par le fait que les longueurs des diagonales ne dépassent pas 33 mm.

6. Filtre selon la revendication 2, caractérisé par le fait que la surface dans laquelle s'étend le réseau est plane, courbe ou présente des plis.

7. Filtre selon la revendication 1, caractérisé par le fait que lesdites figures (11) sont des figures à trois dimensions dont les côtés ont des longueurs comprises entre 4 et 33 mm.

8. Filtre selon la revendication 1, caractérisé par le fait qu'il comprend au moins un élément de connexion (12,13).

9. Filtre selon la revendication 1, caractérisé par le fait que ledit réseau (10) est monté sur un support ou incorporé à la masse d'un corps transparent ou non.

10. Filtre selon la revendication 1, caractérisé par le fait qu'il comprend des moyens permettant de mettre le réseau (10) à terre ou de le relier à une source de tension.

11. Filtre selon la revendication 1, caractérisé par le fait que ledit réseau (10) est constitué par un circuit imprimé.

Fig 1

Fig.2

10                                                          11

10                    Fig.3                                 11

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | GB-A-2 064 629  (L. WIRTZ)<br>* Résumé; page 1, lignes 88-92; page 2, lignes 67-69; revendications 1,2; figures 1,2 *<br>--- | 1-5,8-10 | G 21 K    3/00<br>H 05 K    9/00<br>A 61 N    1/16<br>H 01 Q   15/00 |
| X | DE-A-3 510 212  (R. WIGGENHAUSER)<br>* Résumé; page 11, lignes 6-13; figure 1 *<br>--- | 1,2,6 | |
| X | FR-A-1 010 989  (M. AUDOUZE)<br>* Page 1, colonne de gauche, lignes 1-23; revendications 1,2; figures 1-5 *<br>--- | 1-6,8-10 | |
| X | FR-A-1 107 318  (C. LANGE)<br>* Revendication 1; figures 1-3 *<br>----- | 1-3,8-10 | |

DOMAINES TECHNIQUES RECHERCHES (Int. Cl.4)

A 61 N
G 21 K
H 01 Q
H 05 K

**Le présent rapport a été établi pour toutes les revendications**

| Lieu de la recherche | Date d'achèvement de la recherche | Examinateur |
|---|---|---|
| LA HAYE | 26-05-1989 | WINKELMAN,A.M.E. |